# EUROPEAN PATENT APPLICATION

(11) **EP 3 659 496 A1**
(43) Date of publication of application: **03.06.2020**
(21) Application number: 18209198.3
(22) Date of filing: 29.11.2018
(51) Int. Cl.: A61B 5/00, A61B 5/0482, A61B 5/0484, A61B 5/16

(54) **SELECTION OF SENSORY STIMULI FOR NEUROFEEDBACK TRAINING**

(71) Applicant: Koninklijke Philips N.V., 5656 AG Eindhoven (NL)
(72) Inventor: FLAESCHNER, Nick, 5656 AE Eindhoven (NL); AARTS, Ronaldus Maria, 5656 AE Eindhoven (NL); BOERNERT, Peter, 5656 AE Eindhoven (NL); EWALD, Arne, 5656 AE Eindhoven (NL); GLEICH, Bernhard, 5656 AE Eindhoven (NL); GRAESSLIN, Ingmar, 5656 AE Eindhoven (NL); VAN EE, Raymond, 5656 AE Eindhoven (NL)
(74) Representative: de Haan, Poul Erik

(57) **Abstract**

The invention provides for a medical instrument (100, 400, 500, 600) comprising an activity measurement system (106) configured for measuring brain activity data (138) from a subject (102). The medical instrument further comprises a stimulus presentation system (108) configured for providing sensory stimulus to the subject. The medical instrument further comprises a memory (130) for storing machine executable instructions (132) and for storing a stimulus reinforcer database (134). The stimulus reinforcer database comprises entries. Each entry comprises commands configured for controlling the stimulus presentation system to provide the sensory stimulus to the subject. The medical instrument further comprises a processor (120) for controlling the medical instrument. Execution of the machine executable instructions causes the processor to: control (200) the stimulus presentation system with a set of entries (136) selected from the stimulus reinforcer database to repeatedly provide sensory stimulus to the subject; control (202) the activity measurement system for performing the measurement of the brain activity data during each sensor stimulus; select (204) a chosen entry (140) from the set of entries using the brain activity data; and store (206) the chosen entry in the memory.

## Description

### FIELD OF THE INVENTION

The invention relates to neurofeedback training

### BACKGROUND OF THE INVENTION

In Neurofeedback training brain activity is a type of biofeedback training where brain activity is monitored and used to control sensory stimuli. Neurofeedback training can be used to teach a subject self-regulation of their brain function.

International patent application WO 2017/1032208 discloses a computer-implemented method for biofeedback training of a subject, said method comprising iteratively obtaining a series of biomarkers, each biomarker being representative of a bio-signal of the subject on a first time window; computing an intermediate threshold based on the series of biomarkers on a second time window, such that said intermediate threshold on said second time window could provide the subject with an expected reward ratio; computing a threshold as the weighted sum of the intermediate threshold and the threshold of the previous iteration and reporting in real-time a reward to the subject based on the difference between the biomarker and the computed threshold, wherein at each iteration the time windows are moved forward in time. The invention also relates to a system for implementing the method of the invention.

### SUMMARY OF THE INVENTION

The invention provides for a medical instrument, a computer program product, and a method in the independent claims. Embodiments are given in the dependent claims.

Neurofeedback training is proven efficient for the treatment of cognitive abilities and various psychiatric disorders. In prior art, in the training sessions, desired patterns of, e.g., brain activity are rewarded with single sensory visual, auditory or tactile rewards. However, in prior art these rewards are not chosen in a patient and disease specific way. Embodiments may provide for a means of improving Neurofeedback training by presenting various stimuli and measuring their effect on brain activity.

We propose a system, which is capable of selecting suitable stimuli based on one or more of the following:
1 presenting a multisensory combination of a variety of stimuli (present a set of entries from a stimulus reinforce database),
2 measuring the patient's brain response to the stimuli in predefined neural regions (measuring brain activity data) and
3 an algorithm which optimizes the sensory stimulation based on those measurements (choose a chosen entry from the set of entries using the brain activity data).

In one aspect the invention provides for a medical instrument that comprises an activity measurement system configured for measuring brain activity data from a subject. The medical instrument further comprises a stimulus presentation system configured for providing sensory stimulus to the subject. The stimulus presentation system may for example provide sensory stimulus to one or more senses of the subject. The medical instrument further comprises a memory for storing machine-executable instructions and for storing a stimulus reinforcer database. The stimulus reinforcer database comprises entries. Each entry comprises commands configured for controlling the stimulus presentation system to provide the sensory stimulus to the subject. Alternatively, the stimulus reinforcer database could be described as containing entries which contain commands to provide specific or different sensory stimulus to a subject.

The medical instrument further comprises a processor for controlling the medical instrument. Execution of the machine-executable instructions causes the processor to control the stimulus presentation system with a set of entries from the stimulus reinforcer database to repeatedly provide sensory stimulus to the subject. The set of entries could for example be pre-chosen stimulus reinforcers which are desired to be tested. Execution of the machine-executable instructions further causes the processor to control the activity measurement system for performing the measurement of the brain activity data during each sensor stimulus. Execution of the machine-executable instructions further causes the processor to select a chosen entry from the set of entries using the brain activity data. Execution of the machine-executable instructions further causes the processor to store the chosen entry in the memory.

The execution of the machine-executable instructions causes the medical instrument to present a number of different sensory stimuli to the subject. During the presentation of these sensory stimuli the brain activity is measured. Depending upon the brain activity data then a particular entry or entries may be chosen. This chosen entry is then stored in the memory for future use. This may for example have the advantage of being used for determining the best stimulus to use for a subject when performing neurofeedback.

In another embodiment the sensory stimulus comprises an animation. This may for example be beneficial because the response of a subject to a particular animation may be recorded. This may be useful for choosing an optional animation for neurofeedback training.

In another embodiment the sensory stimulus comprises an animated display of a thermometer, an expanding and contracting circle, a thermometer or a circle which expands and an animation of a bird which either flies or does not fly depending upon particular brain activity or brain state. All of these may for example be examples of an animation which may be tested using the measurement of the brain activity.

In another embodiment the sensory stimulus further comprises a display of an image representing a reward, a generation of a tone as a reward, and a generation of a chosen melody as a reward. All of these may be useful as rewards which are displayed under certain conditions during neurofeedback training. The measurement of the brain activity during the presentation of any of these may be used for quantitatively measuring its effect on the subject's brain activity.

In another embodiment execution of the machine-executable instructions further causes the processor to receive neurofeedback training instructions. The neurofeedback training instructions may for example be instructions which result in a particular training plan or set of instructions for performing individualized neurofeedback for a subject. Execution of the machine-executable instructions further causes the processor to modify the neurofeedback training instructions by incorporating commands from a chosen entry. This for example maybe useful for implementing stimuli which will have the largest effect on a particular subject during neurofeedback training.

Execution of the machine-executable instructions further causes the processor to present a neurofeedback training element to the subject by controlling the stimulus presentation system with the modified neurofeedback training instructions. In this example the neurofeedback or portions of it are presented to the subject and they have been customized using the chosen entry to provide sensory feedback which will be effective for the subject. This may for example result in the increased efficiency or result of neurofeedback training.

In another embodiment the activity measurement system further comprises a respiration measuring system for acquiring respiration data descriptive of a respiration state of the subject. The stimulus reinforcer database further contains respiration indicator entries comprising commands configured for controlling the stimulus presentation system for indicating a desired breathing phase or desired breathing rate of the subject.

Execution of the machine-executable instructions further causes the processor to present the respiration indicator to the subject during the controlling of the stimulus presentation with a set of entries from the database to repeatedly provide sensory stimulus to the subject. Execution of the machine-executable instructions further causes the processor to control the respiration measuring system for performing the acquisition of the respiration data during the controlling of the stimulus presentation with the set of entries from the database to repeatedly provide sensory stimulus to the subject.

Execution of the machine-executable instructions further causes the processor to store a choice of a respiration indicator entry from the respiration indicator entries using the representation data. This embodiment may be beneficial because the measurement of the effectiveness of the respiration indicator is performed at the same time as the measurement of the brain activity in response to the set of entries. This may have the benefit that the choice of the respiration indicator entry has a synergistic reinforcement with the chosen entry.

The respiration indicator may be used during the execution or presenting of the neurofeedback training element to the subject. This may result in a greater effectiveness of the neurofeedback training element.

In another embodiment the breathing indicator comprises any one of the following: an animation that is controlled using the respiration data, an animation of an expanding and contracting circle that is controlled with the respiration data to match a breathing phase of the subject, and a sinusoidal tone whose frequency is changed using the respiration data.

In another embodiment the stimulus presentation system is configured for providing stimulus to more than one sense simultaneously. This may be beneficial because the presentation of more than one stimulus to different senses may have a cooperative effect on increasing the effectiveness of the neurofeedback training.

In another embodiment the stimulus presentation system comprises a visual display. This for example may be a screen or other indicator which can be controlled by a processor.

In another embodiment the stimulus presentation system comprises a virtual reality headset. This may be beneficial because it may be able to provide more complicated or more detailed stimulus to a subject which is more realistic.

In another embodiment the stimulus presentation system comprises an audio speaker, and/or a subwoofer. The inclusion of these may enable the generation of audio stimulus.

In another embodiment the stimulus presentation system comprises a tactile feedback system. The tactile feedback system may for example be a device which provides the stimulus of the touch sensation. This may have a device that either touches, tickles or otherwise stimulates a region of skin of the subject.

In another embodiment the stimulus presentation system comprises a heater and/or a cooler for controlling a temperature that the subject is exposed to at least on a portion of their body.

In another embodiment the stimulus presentation system comprises an instruction display for providing manual tactile feedback instructions. This for example may be useful for a trainer who is manually providing tactile feedback to a subject during a neurofeedback training.

In another embodiment the neurofeedback entry is chosen using a neural network. This may be beneficial because very complicated patterns may be trained instead of being hardwired or programmed. For example, a convolutional neural network could be used and so-called deep learning could be used.

For example, the convolutional neural network could be used to find similar stimuli, so the neural network would act as a recommendation engine that provides suggested stimuli in response to the brain activity data measured for the stimuli caused by controlling the stimulus presentation system with the set of entries.

In another embodiment the neurofeedback entry is chosen using a predetermined criterion. For example, certain activities of the brain within certain regions of the subject maybe above certain levels.

In another embodiment the neurofeedback entry is chosen using a set of rules to determine which is chosen.

In another embodiment the neurofeedback entry is chosen such that the brain activity of the subject is maximized in response to a particular sensory stimulus. This may be helpful in maximizing the effectiveness of the individualized neurofeedback training.

In another embodiment the brain activity measurement system comprises a magnetic resonance imaging system. This may be beneficial because it may be useful for directly measuring brain activity within the brain within the subject.

In another embodiment the magnetic resonance imaging system is configured for measuring the brain activity from the subject using any one of the following: the blood oxygen level dependent functional magnetic resonance imaging measurements from a region of interest and also from measuring neural activity in the amygdala. Both of these methods may provide excellent means of determining the brain activity of a subject quantitatively.

In another embodiment the stimulus presentation system comprises active noise cancelling headphones configured for providing audio stimulus to the subject. This for example may be useful in a magnetic resonance imaging system because during the generation of magnetic gradient pulses large knocking noises may be generated. The use of the active noise cancelling headphones may be used to both provide the audio stimulus and also reduce distracting audio stimulus at the same time.

In some examples the acoustic stimulus is chosen to be louder than the noise cancelled noise of the gradient fields.

In another embodiment the brain activity measurement system comprises an EEG system (an electroencephalography system). The use of this EEG system may be beneficial because it may provide a very effective and quantitative measurement of brain activity at reasonable cost. The EEG system may also be integrated into a magnetic resonance imaging system providing a better and more accurate measurement of the brain activity of a subject.

In another embodiment the brain activity measurement system comprises a magnetoencephalography system. This may be beneficial because it is able to directly measure small currents within the subject's brain.

In another aspect the invention provides for a computer program product comprising machine-executable instructions for execution by a processor controlling the medical instrument. The medical instrument comprises an activity measurement system configured for measuring brain activity data from a subject and a stimulus presentation system configured for providing sensory stimulus to the subject. Execution of the machine-executable instructions causes the processor to control the stimulus presentation system with a set of entries from a stimulus reinforcer database to repeatedly provide sensory stimulus to the subject. The stimulus reinforcer database comprises entries.

Each entry comprises commands configured for controlling the stimulus presentation system to provide the sensory stimulus to the subject. Execution of the machine-executable instructions further causes the processor to control the activity measurement system for performing the measurement of the brain activity data during each sensor stimulus. Execution of the machine-executable instructions further causes the processor to select a chosen entry from the set of entries using the brain activity data. Execution of the machine-executable instructions further causes the processor to store the chosen entry in the memory. The advantages of this have been previously discussed.

In another aspect the invention provides for a method of operating a medical instrument. The medical instrument comprises an activity measurement system configured for measuring brain activity data from a subject and a stimulus presentation system configured for providing sensory stimulus to the subject. The method comprises controlling the stimulus presentation system with a set of entries from a stimulus reinforcer database to repeatedly provide sensory stimulus to the subject.

The stimulus reinforcer database comprises entries wherein each entry comprises commands configured for controlling the stimulus presentation system to provide the sensor stimulus to the subject. The method further comprises controlling the activity measurement system for performing the measurement of the brain activity data during each sensor stimulus. The method further comprises selecting a chosen entry from the set of entries using the brain activity data. The method further comprises storing the chosen entry in the memory.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins using the antenna of a Magnetic resonance apparatus during a magnetic resonance imaging scan. MRF magnetic resonance data is magnetic resonance data. Magnetic resonance data is an example of medical image data. A Magnetic Resonance Imaging (MRI) image or MR image is defined herein as being the reconstructed two- or three-dimensional visualization of anatomic data contained within the magnetic resonance data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical instrument;
Fig. 2 shows a flow chart which illustrates a method of using the medical instrument of Fig. 1;
Fig. 3 shows a flow chart which illustrates a further method of using the medical instrument of Fig. 1;
Fig. 4 illustrates a further example of a medical instrument;
Fig. 5 illustrates a further example of a medical instrument;
Fig. 6 illustrates a further example of a medical instrument;
Fig. 7 illustrates a implementation of a system for generating modified neurofeedback training instructions;
Fig. 8 illustrates an example of an animation of a thermometer;
Fig. 9 shows an animation of an expanding and/or contracting sphere;
Fig. 10 shows an animation of a flying bird;
Fig. 11 illustrates a number of rewards which can be presented to a subject during neurofeedback training;
Fig. 12 illustrates an example of a visual breathing indicator; and
Fig. 13 illustrates an example of an audible breathing indicator.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

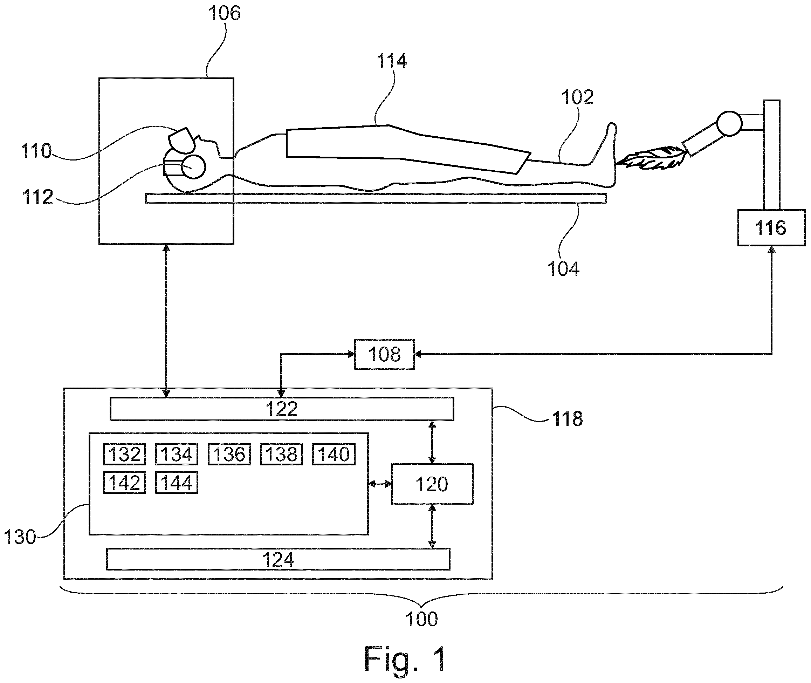

Fig. 1 illustrates an example of a medical instrument 100. The medical instrument is shown as containing a subject support 104 which supports a subject 102. The medical instrument 100 is further shown as comprising an activity measurement system 106 that is configured for measuring brain activity data from the subject 102. The activity measurement system may be any system which is used to quantitatively measure the brain function or amount of brain activity of the subject 102. Several examples of this may be a magnetic resonance imaging system, an EEG system, and a MEG system. In some instances, the magnetic resonance imaging system and the EEG system could both be combined. The medical instrument 100 is further shown as containing a stimulus presentation system 108.

The stimulus presentation system may comprise elements which are able to present various stimuli to one or more senses of the subject 102. Fig. 1 illustrates several different examples. The subject 102 is shown as wearing a pair of virtual reality goggles 110. As an alternative to virtual reality goggles there may be a screen or display which is visible to the subject 102. The subject 102 is also shown as wearing headphones 112. The headphones may be used to present an audio stimulus to the subject 114. An alternative to the headphones 112 would be the use of speakers and/or subwoofers. The use of headphones 112 maybe particularly beneficial because they may be noise cancelling headphones and may be useful in reducing extraneous stimuli also. The stimulus presentation system 108 is further shown as comprising a body temperature control system 114.

The body temperature control system may for example be used to raise or lower an external temperature the subject 102 is exposed to. The stimulus presentation system 108 is further shown as containing a tactile feedback system 116. This for example may be used for providing a tactile stimulus to the subject 102. As an alternative to a tactile feedback system 116 there may be a display which displays instructions to a helper which manually provides tactile feedback to the subject 102. Connections between the stimulus presentation system 108 and the various elements of it are not displayed. These may be either wired and/or wireless connections.

The medical instrument 100 is further shown as comprising a computer 118. The computer comprises a processor 120. The processor 120 is intended to represent one or more processors that may be within the same computing device or may even be distributed amongst various locations or different computing devices. The processor 120 is in communication with a hardware interface 122 that enables the processor 120 to control the other components of the medical instrument 100. The processor 120 is further in communication with an optional user interface 124 that may be used for displaying and/or receiving data from a subject. The processor 120 is further in communication with a memory 130. The memory 130 may be any memory which is accessible to the processor 120. It may for example be volatile or non-volatile memory and may also represent a storage medium such as a hard drive or optical disc. The memory 130 is shown as containing machine-executable instructions 132.

Execution of the machine-executable instructions 132 by the processor 120 enable it to control the operation and function of the medical instrument 100. The machine-executable instructions 132 may also enable the processor 120 to perform various data processing and image processing tasks. The memory 130 is further shown as containing a stimulus reinforcer database 134. The stimulus reinforcer database 134 comprises entries. Each entry comprises commands configured for controlling the stimulus presentation system to provide the sensory stimulus to the subject. The stimulus reinforcer database 134 may be thought of as a collection of possible stimuli which may be presented to the subject 102. The activity measurement system 106 is then used to measure the subject's response 102 to the various stimulus of the stimulus reinforcer database 134 by measuring the brain activity of the subject. The memory 130 is shown as containing a set of entries 136 selected from the stimulus reinforcer database 134.

These for example may be a predetermined set of entries 136 or may be selected on the fly using a set of rules or other conditions. Each of the set of entries 136 is then used to control the stimulus presentation system 108 and simultaneously brain activity data 138 is acquired. The brain activity data 138 is then a quantitatively measured response of the subject 102 to each of the set of entries 136. The memory 130 is further shown as containing a chosen entry 140. The chosen entry 140 may for example be one of the set of entries 136 that was selected using the brain activity data 138. A set of predetermined criteria or other selection criteria may be used to select the chosen entry 140. Due to the brain activity data 138 the chosen entry 140 can be used to optimize the subject's response during for example neurofeedback training.

The memory 130 is further shown as containing optional neurofeedback training instructions 142. The neurofeedback training instructions 142 are instructions which control the medical instrument 100 to control the stimulus presentation system 108 such that the subject 102 is trained using neurofeedback techniques. The memory 130 is further shown as containing modified neurofeedback training instructions 144. The modified neurofeedback training instructions 144 were modified using the chosen entry 140. For example, the optional neurofeedback training instructions 142 may be constructed such that they are generally applicable or usable for subjects in general. However, the brain activity data 138 was used to select a particular stimulus that the subject 102 responded to. This may then be used to optimize or improve and create the modified neurofeedback training instructions 144.

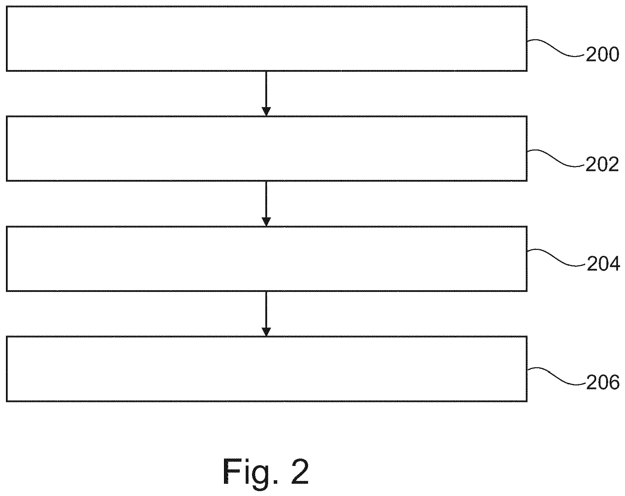

Fig. 2 shows a flowchart which illustrates a method of operating the medical instrument 100 of Fig. 1. First in step 200 the stimulus presentation system 108 is controlled with the set of entries 136 from the stimulus reinforcer database 134 to repeatedly provide sensory stimulus to the subject 102. Next in step 202 the activity measurement system 106 is controlled to acquire the brain activity data 138 during each sensor stimulus which is caused by each of the set of entries. Next in step 204 a chosen entry 140 is selected from the set of entries 136 using the brain activity data 138. Finally, in step 206 the chosen entry 140 is stored in the memory 130 for further use.

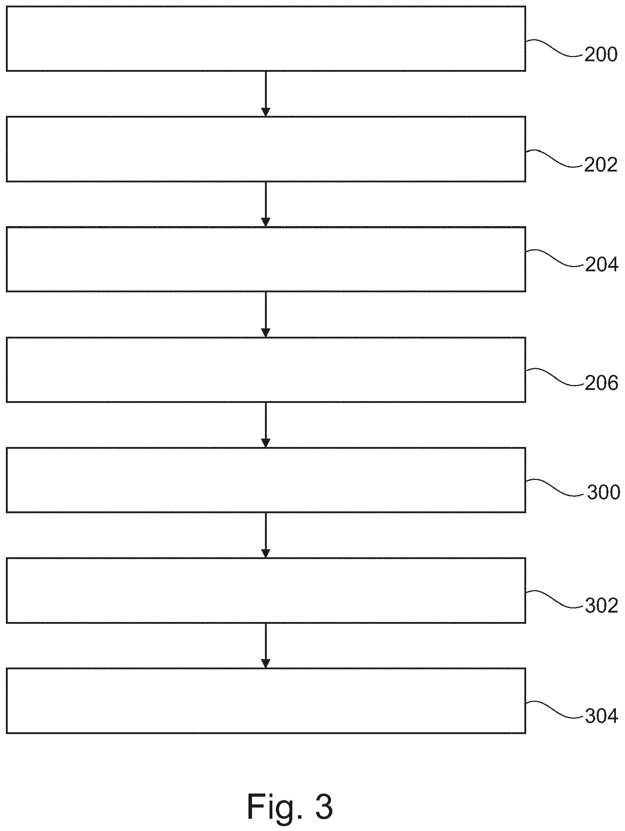

Fig. 3 shows a figure which illustrates a further method of operating the medical instrument 100 shown in Fig. 1. In Fig. 3 the method starts out with the first four steps of Fig. 2. After steps 200-206 have been performed the method proceeds to step 300. In step 300 the neurofeedback training instructions 300 are received. Next in step 302 the neurofeedback training instructions are modified by incorporating commands from the chosen entry 140. And finally, in step 304, the stimulus presentation system is controlled by executing the modified neurofeedback training instructions 144.

It should be noted that during the execution of the modified neurofeedback training instructions 144 additional brain activity data 138 can be acquired. This for example could be used to measure the effectiveness of the neurofeedback training instructions as they are used. For example if it is found that the neurofeedback training instructions are losing their effectiveness the chosen entry 140 can be switched for example by going through and repeating the process of screening all of the set of entries using the brain activity data 138 or for example, if the brain activity data indicates that there are several highest or several more effective ways of stimulating the subject 102 the chosen entry 140 can be switched. This may even be performed on the fly.

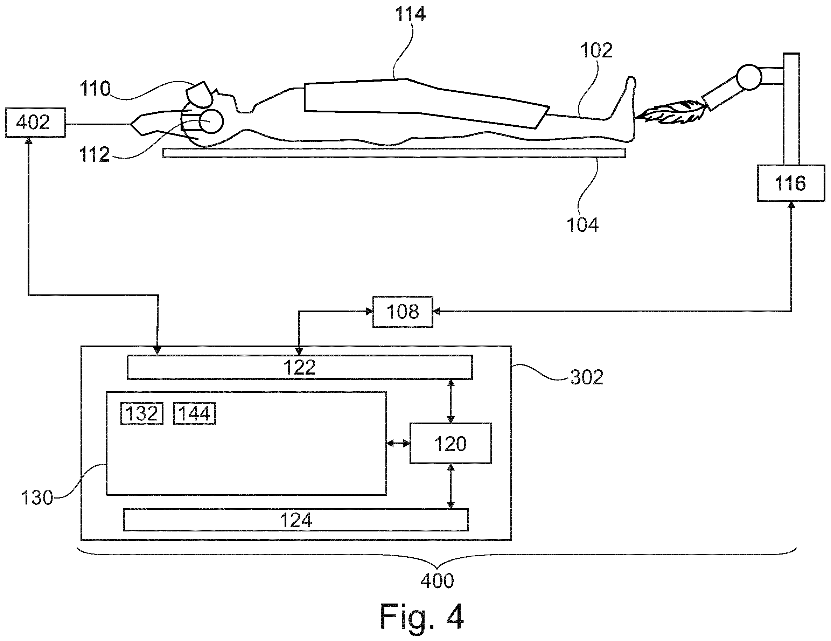

Fig. 4 illustrates a further example of a medical instrument 400. The medical instrument 400 of Fig. 4 is different than the one depicted in Fig. 1. In this example the medical instrument 400 is shown as comprising an EEG system 402. The EEG system 402 is the activity measurement system 106. If the activity measurement system 106 comprises an MRI system or an MEG system it may be expensive or difficult to provide to all training locations. The subject 102 could for example be sent to a central examining station or area where the medical instrument 100 of Fig. 1 comprises an MEG system or an MRI system. The modified neurofeedback training instructions 144 can then be transferred to a different medical instrument 400. The memory 130 is shown as containing the modified neurofeedback training instructions 144. The neurofeedback training can then be performed using the more cost effect EEG system 402.

The medical instrument 400 depicted in Fig. 4, could also be able to perform the methods depicted in Figs. 1 and 2 using the EEG system. However, Fig. 4 illustrates that once the chosen entry, which indicates an effective stimulus that can be used for a particular subject, is determined it can be transported to other locations and used with other hardware configurations. This could for example be integrated into patient records or data carriers that the subject brings to training.

Because the choice of the chosen entry is transportable the overall medical instrument could in some examples considered to be a combination of the instruments shown in Fig. 1 and Fig. 4. The various components may therefore be in some instances distributed.

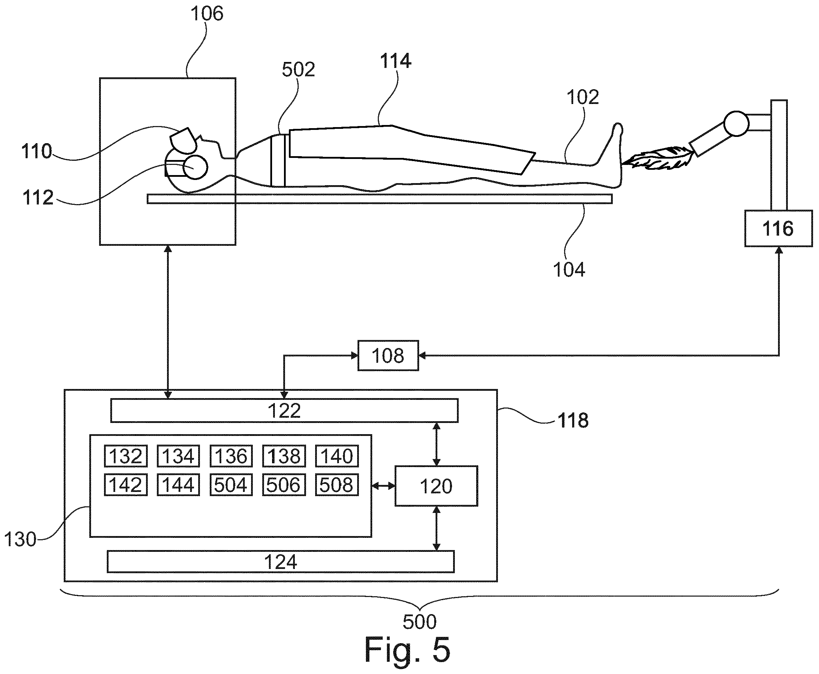

Fig. 5 illustrates a further example of a medical instrument 500. The medical instrument 500 of Fig. 5 is similar to the medical instrument 100 of Fig. 1 except there is now additionally a respiration belt 502. The respiration belt 502 is able to send a signal as the subject 102 expands and contracts his or her thorax during breathing. The respiration belt 502 is an example of a respiration measurement system. The respiration belt 502 acquires respiration data 506. The memory 130 is further shown as containing respiration indicator entries 504. For example, the audio 112, the visual 110 or the tactile 116 could be used to indicate a breathing rate or phase that is desired to the subject 102. The respiration indicator entries 504 may provide different ways of presenting this to the subject 102.

For example, the position or expansion of an animation or other object in the visual system 110 may be used to represent a respiration phase. The respiration indicator entries 504 provide several different possibilities which can be tested directly on the subject 102. During the measurement of the brain activity data 138 the respiration data 506 can also be recorded and stored in the memory 130. Various respiration indicator entries 504 can be presented with the different choices presented by the set of entries 136. When the chosen entry 140 is selected a chosen respiration indicator 508 from the respiration indicator entries 504 can be selected using the respiration data 506. The control of the subject's 102 respiration using the chosen respiration indicator 508 may help with the effectiveness of neurofeedback training. The presentation of various respiration indicators at the same time as the different stimulus reinforcers indicated by the set of entries may help to select a means of presenting the respiration phase or rate to the subject 102 that is most compatible with the chosen entry 140.

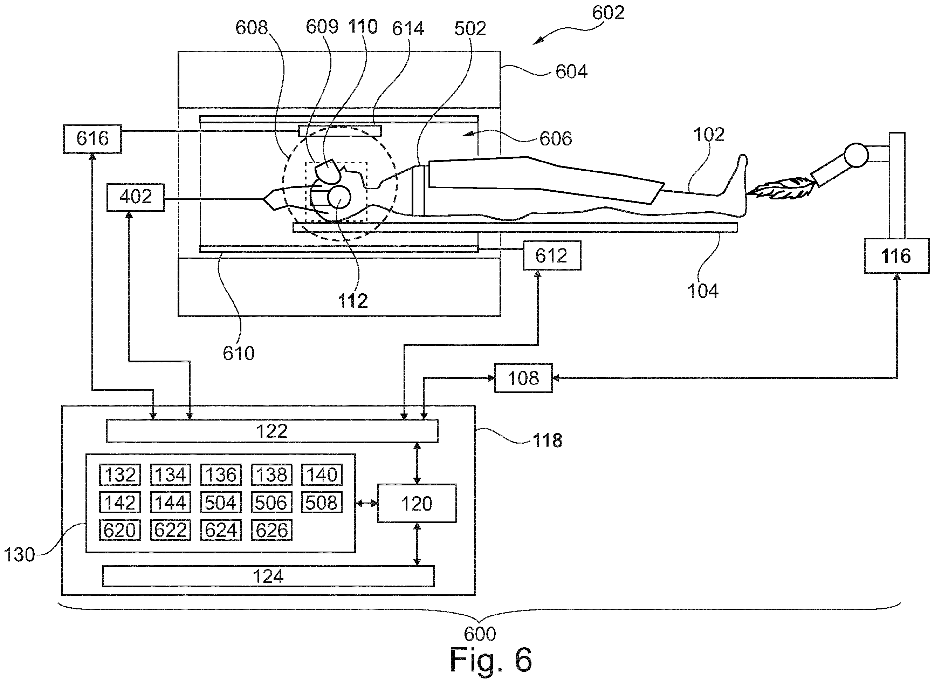

Fig. 6 illustrates a further example of a medical instrument 600. In this example the activity measurement system 106 comprises an EEG system 402 and a magnetic resonance imaging system 602. Although the EEG 402 and the magnetic resonance imaging system 602 are both depicted in Fig. 6 the medical instrument 600 could be constructed also with just the EEG system 402 and just the magnetic resonance imaging system 602. However, the EEG system 402 and the magnetic resonance imaging system 602 acquire complimentary data which can be used to improve the quality of the chosen entry 140.

The magnetic resonance imaging system 602 comprises a magnet 604. The magnet 604 is a superconducting cylindrical type magnet with a bore 606 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 606 of the cylindrical magnet 604 there is an imaging zone 608 where the magnetic field is strong and uniform enough to perform magnetic resonance imaging. A region of interest 609 is shown within the imaging zone 608. The magnetic resonance data that is acquired typically acquried for the region of interest. A subject 102 is shown as being supported by a subject support 104 such that at least a portion of the subject 102 is within the imaging zone 608 and the region of interest 609.

Within the bore 606 of the magnet there is also a set of magnetic field gradient coils 610 which is used for acquisition of preliminary magnetic resonance data to spatially encode magnetic spins within the imaging zone 608 of the magnet 604. The magnetic field gradient coils 610 connected to a magnetic field gradient coil power supply 612. The magnetic field gradient coils 610 are intended to be representative. Typically magnetic field gradient coils 610 contain three separate sets of coils for spatially encoding in three orthogonal spatial directions. A magnetic field gradient power supply supplies current to the magnetic field gradient coils. The current supplied to the magnetic field gradient coils 610 is controlled as a function of time and may be ramped or pulsed.

Adjacent to the imaging zone 608 is a radio-frequency coil 614 for manipulating the orientations of magnetic spins within the imaging zone 608 and for receiving radio transmissions from spins also within the imaging zone 608. The radio frequency antenna may contain multiple coil elements. The radio frequency antenna may also be referred to as a channel or antenna. The radio-frequency coil 614 is connected to a radio frequency transceiver 616. The radio-frequency coil 614 and radio frequency transceiver 516 may be replaced by separate transmit and receive coils and a separate transmitter and receiver. It is understood that the radio-frequency coil 614 and the radio frequency transceiver 516 are representative. The radio-frequency coil 614 is intended to also represent a dedicated transmit antenna and a dedicated receive antenna. Likewise the transceiver 616 may also represent a separate transmitter and receivers. The radio-frequency coil 614 may also have multiple receive/transmit elements and the radio frequency transceiver 616 may have multiple receive/transmit channels. For example if a parallel imaging technique such as SENSE is performed, the radio-frequency could 614 will have multiple coil elements.

In this example the subject, 102 is positioned such that the subject's head region is within the region of interest 609 so that measurements on the brain can be made.

The stimulus presentation system 108, the activity measurement system 106, the transceiver 616, and the gradient controller 612 are shown as being connected to a hardware interface 122 of the computer system 118. The memory 130 is further shown as containing pulse sequence commands 620. The pulse sequence commands enable the processor 120 to acquire magnetic resonance data 622 according to a magnetic resonance imaging protocol. The magnetic resonance imaging protocol may be selected so that it is able to measure brain activity of the subject 102 directly.

For example, the magnetic resonance imaging protocol could be selected so that the so-called BOLD response is measured in real time using functional magnetic resonance imaging. The memory 130 is shown as containing magnetic resonance data 622 that was acquired by executing the pulse sequence commands 620. The memory 130 is further shown as containing a magnetic resonance image 624 that was reconstructed from the magnetic resonance data 622. The magnetic resonance image 624 could for example show the brain activity of the subject 102 at different periods of time. The memory 130 is further shown as containing EEG data 626 that was acquired using the EEG system 401. The magnetic resonance image 624 and the EEG data 626 could be used to provide the brain activity data 138.

Neurofeedback training is a promising approach for treating a large variety of psychiatric diseases/disorders, e.g. ADHD, ADD, depression, autism, anxiety disorders, bipolar disorders, dementia, and many more. Besides treating diseases/disorders, neurofeedback training can also improve the cognitive performance of healthy subjects. During a training session, the patient's brain activity is constantly monitored in real-time using, e.g., fMRI, EEG or MEG. The patient tries to reach a desired state of mind, e.g., happiness or focus. When this desired pattern of brain activity is reached, a visual, auditory or tactile reward/reinforcer (the feedback) is presented to the patient. After repeated training (typically >10 sessions) the patient is supposed to be able to reach the desired state of mind without the feedback signal.

A current problem of neurofeedback training is that the reinforcers (sensory stimuli) are not selected individually. Thus, they lack specificity with respect to subject and disease specific characteristics. For example, indications were found that learning-disabled children benefit more from auditory than from visual reinforcers while in clinical practice, typically visual reinforcers are used.

Examples may provide for a system, which is capable of selecting the optimal reinforcer (chosen element from a stimulus reinforce database) in a patient (subject) and/or disease specific manner. The system may comprise a database (stimulus reinforce database 134) of possible stimuli, an apparatus, which can present the stimuli to the patient, and a measurement device (activity measurement system 106), which measures the patient's response to the different stimuli (brain activity 138). Finally, an algorithm chooses the optimum stimuli (choice of the chosen entry 140) based on the measurement results and based on a priori knowledge with respect to patient and/or disease specific characteristics, after a sufficient amount of data exists.

First, a database of possible stimuli is provided. Possible stimuli are of
- visual (e.g., pictures and videos of various objects and with variable degree of detail etc.),
- auditory (ranging from simple sinus tones to various noises to complex music) and
- tactile (e.g., synthetic jets)
nature or combinations thereof. In one embodiment, the stimulus database is provided on a computer.

Second, a system (stimulus presentation system) which is capable of presenting the stimulus to the patient is provided.

In one example, visual and auditory stimuli are provided to the patient using a screen and headphones.

In another example, the stimuli are presented in a virtual reality environment to the patient.

In another example tactile stimuli can be provided both manually (by, e.g., a practitioner who gets informed when the stimulus is to be applied and which stimulus has to be provided) or automatically by, e.g., computer-controlled devices such as synthetic jets, systems which can provide heat / cold, or mechanical systems which can, e.g. poke the patient.

In another example, multisensory combinations of stimuli are employed. To be more precise: it is known from literature that multisensory rhythmically synchronous signals create a synergetic attentional capture effect across the used senses (1+1>2), while multisensory non-synchronous signals are perceived as annoying. The attentional capture of synchronous multisensory signals helps the patient to keep focusing on the task at hand for neurofeedback. In this embodiment one must be particularly careful that the periods of the stimuli that are to be combined are identical (the deviation should be within the order of milliseconds after a ten-minutes trial run), which can be ensured by using one single signal generating apparatus that is connected to the stimuli generating apparatuses (earphones, screen, tactile device etc.).

In another example, the participant is exposed to the neurofeedback stimuli after lowering the breathing rate using device-guided breathing to help the patient to keep focused attention on the exposed stimuli. It is known from literature that that a lower breathing rate during exposure training facilitates the capability of the patient to undergo effective exposure. With lower breathing rate a patient experiences a greater sense of control and thereby facilitating the positive outcome effect of the exposure and the combined neurofeedback. Lower breathing rate is believed to reduce the arousal level and excitability of sympathetic "fight-flight" behaviors.

The system may also comprise a measurement system (an activity measurement system), which is capable of measuring the patient's response to those stimuli, is provided. Possible modalities include EEG, MEG and fMRI. Possible observables are magnitudes and timings of event related potentials (fields) in EEG (MEG) or BOLD response (fMRI).

In one example, the response of the brain's region of interest for the neurofeedback is being recorded.

In another example neural activity from the amygdala is being recorded (e.g., the BOLD response in real-time fMRI). It is known from literature that the amygdala reflects the emotional (and mood) state associated with the stimulus signal.

In another example, the relative ratio of brain waves (relative power of frequency bands in EEG or MEG) or connectivity patterns (EEG/MEG/fMRI) or other derived quantities are recorded.

In another example, not only the patient's physiological response to the stimulus but also the subsequent task performance (e.g. keeping focus, reaction tests) is recorded.

The system may also comprise, an algorithm (machine executable instructions), possibly an autonomously learning 'artificial intelligent agent'(possibly implemented using a convolutional neural network), is used to select the optimal stimulus.

In one example the optimal stimulus is determined in a calibration session preceding the first training session. During this session, various stimuli are presented to the patient, while the patient's response to those stimuli is constantly monitored as described above. Then, the stimulus with, e.g., the
- strongest response (e.g., largest amplitude of ERP, ERF or BOLD response),
- fastest response,
- best subsequent task performance and/or
- desired change of connectivity patterns
or combinations thereof is chosen for the subsequent training session.

In one example, it is first determined whether a visual, auditory or tactile stimulus or a combination thereof is most suitable and then the most suitable stimulus out of this subset is found.

In another example, the response to the stimulus is also monitored during the training sessions and can be changed online, e.g., in the case of wear-out-effects. Then either a new calibration session is performed, or the second-best stimulus is chosen, or a new stimulus based on the results of the first calibration session and, e.g., a neighborhood-classifier which selects similar stimuli, is chosen. For example, an image classification algorithm could select similar images. The selection algorithm can also be trained to select suitable stimuli for new patients based on a recommendation engine, without the need of a prior calibration session.

In one example, this algorithm takes into account the diagnosis and patient specific characteristics (e.g., age, gender) and has been trained with results of calibration sessions and training outcomes of similar patients.

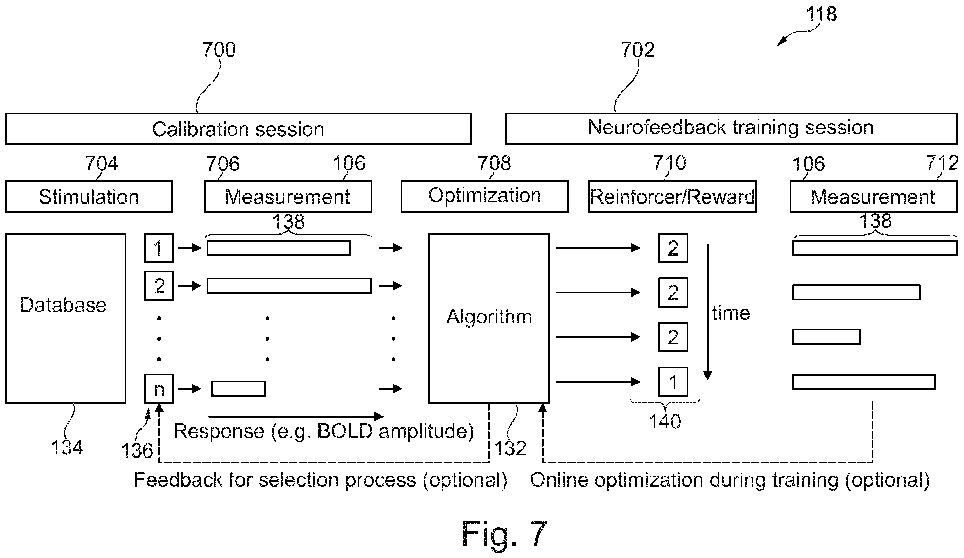

Fig. 7 illustrates an example of a functional implementation of a computer system 118 that is able to generate the chosen entries 140 for performing neurofeedback training. In the example there is a calibration session 700 and then a neurofeedback training session 702. The calibration session 700 is divided into a simulation phase, a measurement phase and an optimization phase 708. The neurofeedback training session optionally has also an optimization phase 708, has a reinforcer reward portion 710 that comprises the neurofeedback training and optionally a measurement phase 712 again. For example new measurements during the measurement phase 712 may be used for further optimization and selection of the chosen entry 140. During the simulation phase 704 the stimulus reinforcer database 134 is used to select the set of entries 136. Then the measurements 706 are performed using the activity measurement system 106. This results in the acquisition of the brain activity data 138. An algorithm 132 is then used to optimize this and select the chosen entries 140 which are used to make the modified neurofeedback training instructions.

In the example of Fig. 7, a variety of different stimuli is presented one after another to the patient (e.g., using a screen and headphones for auditory, visual and multisensory (audio + video) stimuli). After each stimulus presentation, a real-time fMRI measurement reveals the BOLD response at the amygdala, which reflects the emotional state associated with the stimulus signal. The larger the BOLD response, the stronger the stimulus effects the emotional state of the subject. The results (the BOLD response amplitude) are stored and, e.g., sorted by the BOLD response amplitude at the amygdala. For the subsequent neurofeedback training session, initially the stimulus with the larges BOLD response (here: stimulus #2) is chosen by the optimization algorithm. In this example embodiment, the BOLD response at the amygdala is measured via real-time fMRI also during the training session. With time, the response to the stimulus may decrease due to wear-out effects. Once the response has gotten weaker than the response of the second strongest stimulus (here: stimulus #1), the second strongest stimulus is selected for the subsequent task(s). Note that this online optimization during the training session is optional. Also note that the optimization algorithm can optionally be used already during the calibration session for optimizing the selection process. This can be done by, e.g., classifying the available stimuli and proposing the most promising stimuli, after a certain amount of data was collected. E.g., when it is found that the patient apparently responds stronger to detailed photographs compared to abstract patterns, then subsequently more detailed photographs and fewer abstract patterns are shown. This classification could be done manually or by means of an image classification algorithm.

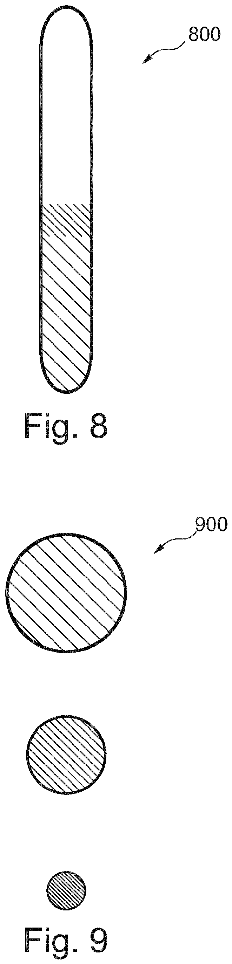

Fig. 8 illustrates an example of an animation 800 of a thermometer. The animation of the thermometer 800 may use different temperatures to indicate different states as measured by the neurofeedback training.

Fig. 9 shows an animation of an expanding and/or contracting sphere 900. This also may be used as a visual neurofeedback stimulus.

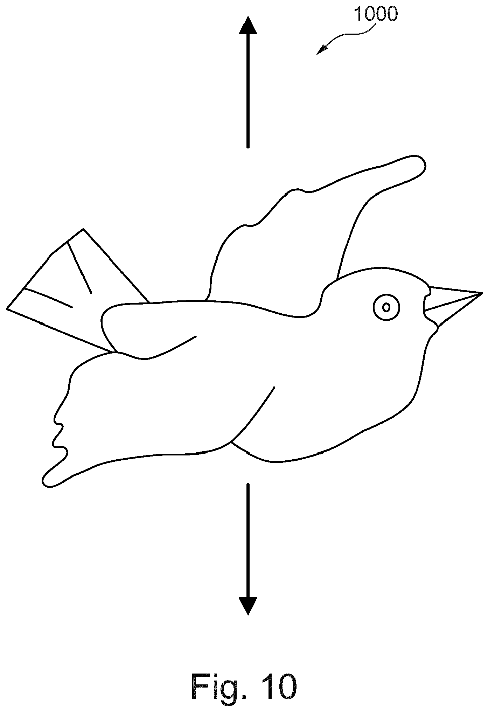

Fig. 10 shows an animation of a flying bird 1000. The position or height of the bird 1000 may for example be used as feedback for neurofeedback training.

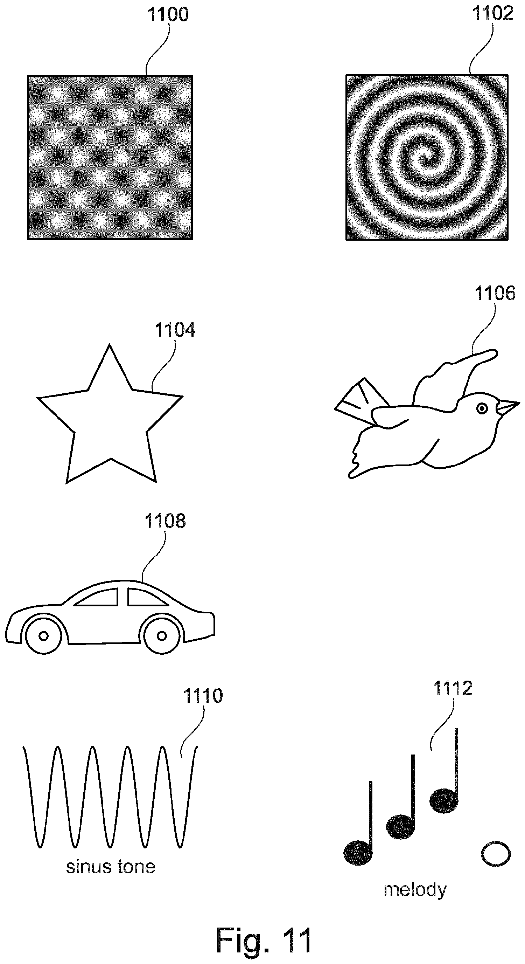

Fig. 11 illustrates a number of rewards which can be presented to a subject during neurofeedback training. For example, Fig. 11 shows a geometric pattern reward 1100 that may be displayed to the subject. Fig. 11 also shows an alternative geometric pattern reward 1102 that may be displayed to the subject. Fig. 11 further illustrates a star reward image 1104 that may be presented to the subject as a reward. Fig. 11 further shows a bird reward image 1106 that may be presented to the subject as a reward. Fig. 11 further illustrates an image of an automobile or car 1108 which may be presented to the subject as a reward. Fig. 11 further shows a representation of a sinusoidal tone 1110 which may be an audible tone reward 1110 that is presented to the subject. Item 1112 represents an audible melody reward 1112 that may also be presented to the subject. The items illustrated in Figs. 8, 9, 10, and 11 may all be inputted in the set of entries 136 that is tested by measuring the brain activity data 138.

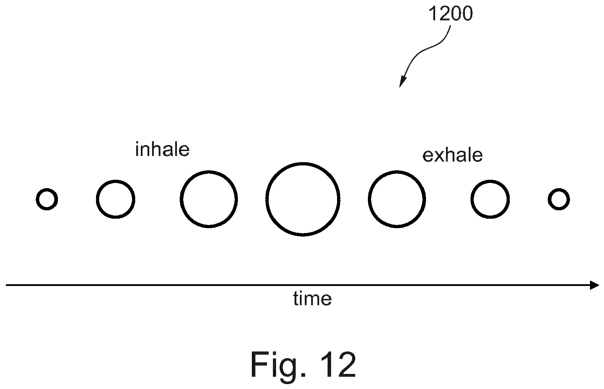

Fig. 12 illustrates an example of a visual breathing indicator 1200. The visual breathing indicator 1200 shows an animation of an expanding and contracting circle over time the size of the circle is intended to indicate the breathing phase of the subject.

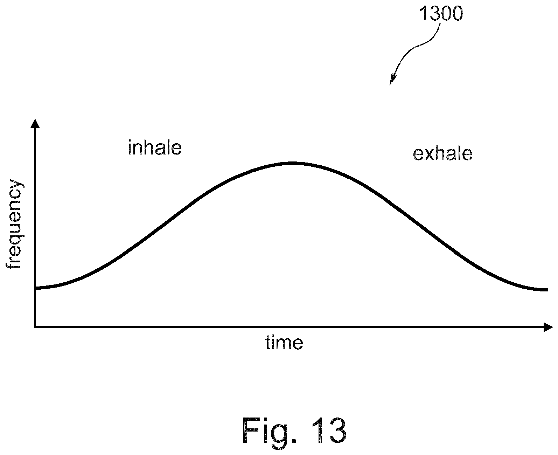

Fig. 13 illustrates an example of an audible breathing indicator 1300. In this case the tone of a sinusoidal tone or other tone is varied as a function of time to indicate the breathing phase of the subject. For example, as the subject inhales the frequency may either increase or decrease.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical instrument
- 102: subject
- 104: subject support
- 106: activity measurement system
- 108: stimulus presentation system
- 110: virtual reality goggles
- 112: headphones
- 114: body temperature control system
- 116: tactile feedback system
- 118: computer
- 120: processor
- 122: hardware interface
- 124: user interface
- 130: memory
- 132: machine executable instructions
- 134: stimulus reinforcer database
- 136: set of entries
- 138: brain activity data
- 140: chosen entry
- 142: neurofeedback training instructions
- 144: modified neurofeedback training instructions
- 200: control the stimulus presentation system with a set of entries from the stimulus reinforcer database to repeatedly provide sensory stimulus to the subject
- 202: control the activity measurement system for performing the measurement of the brain activity data during each sensor stimulus
- 204: select a chosen entry from the set of entries using the brain activity data
- 206: store the chosen entry in the memory
- 300: receive neurofeedback training instructions
- 302: modify the neurofeedback training instructions by incorporating commands from the chosen entry
- 304: control the stimulus presentation system with the modified neurofeedback training instructions
- 400: medical instrument
- 401: EEG system
- 500: medical instrument
- 502: respiration belt (respiration measurement system)
- 504: respiration indicator entries
- 506: respiration data
- 508: chosen respiration indicator
- 600: medical instrument
- 602: magnetic resonance imaging system
- 604: magnet
- 606: bore of magnet
- 608: imaging zone
- 609: region of interest
- 610: magnetic field gradient coils
- 612: magnetic field gradient coil power supply
- 614: radio-frequency coil
- 616: transceiver
- 620: pulse sequence commands
- 622: magnetic resonance data
- 624: magnetic resonance image
- 626: EEG data
- 700: calibration session
- 702: neurfeedback training session
- 704: simulation
- 706: measurement
- 708: optimiation
- 710: Reinforcer / Reward
- 712: Measurement
- 800: thermometer animation
- 900: expanding / contracting sphere animation
- 1000: flying bird animation
- 1100: geometric pattern reward image
- 1102: geometric pattern reward image
- 1104: star reward image
- 1106: bird reward image
- 1108: car image reward
- 1110: audible sinusoidal tone reward
- 1112: audible melody reward
- 1200: visual breathing indicator
- 1300: audible breating indicator

## Claims

1. A medical instrument (100, 400, 500, 600) comprising:
- an activity measurement system (106) configured for measuring brain activity data (138) from a subject (102);
- a stimulus presentation system (108) configured for providing sensory stimulus to the subject;
- a memory (130) for storing machine executable instructions (132) and for storing a stimulus reinforcer database (134), wherein the stimulus reinforcer database comprises entries, wherein each entry comprises commands configured for controlling the stimulus presentation system to provide the sensory stimulus to the subject;
- a processor (120) for controlling the medical instrument, wherein execution of the machine executable instructions causes the processor to:
- control (200) the stimulus presentation system with a set of entries (136) selected from the stimulus reinforcer database to repeatedly provide sensory stimulus to the subject;
- control (202) the activity measurement system for performing the measurement of the brain activity data during each sensor stimulus;
- select (204) a chosen entry (140) from the set of entries using the brain activity data; and
- store (206) the chosen entry in the memory.

2. The medical instrument of claim 1, wherein the sensory stimulus comprises any one of the following: an animation , an animated display of a thermometer (800), and expanding and contracting circle (900), an animation of a bird (1000) which changes a flying state, a display of an image representing a reward (1100, 1102, 1104, 1106, 1108), a generation of a tone as a reward (1110), a generation of a chosen melody (1112) as a reward, and combinations thereof.

3. The medical instrument of claim 1, wherein execution of the machine executable instructions further causes the processor to:
- receive (300) neurofeedback training instructions (142);
- modify (302) the neurofeedback training instructions by incorporating commands from the chosen entry; and
- control (304) the stimulus presentation system with the modified neurofeedback training instructions (144).

4. The medical instrument of claim 1, 2, or 3, wherein the activity measurement system further comprises a respiration measurement system (502) for acquiring respiration data descriptive of a respiration state of the subject, wherein the stimulus reinforcer database further contains respiration indicator entries (504) comprising commands configured controlling the stimulus presentation system for indicating a desired breathing phase or a desired breathing rate to the subject,
wherein execution of the machine executable instructions further causes the processor to:
- present the respiration indicator to the subject during the controlling of the stimulus presentation with the set of entries from the database to repeatedly provide sensory stimulus to the subject;
- control the respiration measurement system for performing the acquisition of the respiration data during the controlling of the stimulus presentation with the set of entries from the database to repeatedly provide sensory stimulus to the subject; and
- store a chosen respiration indicator entry (508) from the respiration indicator entries using the respiration data.

5. The medical instrument of claim 4, wherein the breathing indicator comprises any one of the following: an animiation that is controlled using the respiration data, and animation of an expanding and contracting circle (1200) that is controlled with the resipiration data to match a breathing phase of the subject, and a sinusoidal tone (1300) whose frequency is changed using the respiration data.

6. The medical instrument of any one of the preceding claims, wherein the stimulus presentation system is configured for providing stimulus to more than one sense simultaneously.

7. The medical instrument of any one of the preceding claims, wherein the stimulus presentation system comprises any one of the following: a visual display, a virtual reality headset (110), a headphone (112), an audio speaker, a subwoofer, a tactical feedback system, a heater, a cooler, an instruction display for providing manual tactile feedback instructions, and combinations thereof.

8. The medical instrument of any one of the preceding claims, wherein the neurofeedback entry is chosen using any one of the following:
- a neural network:
- a predetermined criteria;
- a set of rules;
- such that the brain activity of the subject is maximized; and
- combinations thereof.

9. The medical instrument of any one of the preceding claims, wherein the brain activity measurement system comprises a magnetic resonance imaging system (602).

10. The medical instrument of claim 9, wherein the magnetic resonance imaging system is configured for measuring the brain activity data from the subject using any one of the following: blood oxygenation level dependent functional magnetic resonance imaging measurements from a region of interest, and measuring neural activity in the amygdala.

11. The medical instrument of claim 9 or 10, wherein the stimulus presentation system comprises an active noise cancelling headphones (112) configured for providing audio stimulus to the subject.

12. The medical instrument of any one of the preceding claims, wherein the brain activity measurement system comprises an electroencephalography system (402).

13. The medical instrument of any one of claims 1 through 9, wherein the brain activity measurement system comprises a magnetoencephalography system.

14. A computer program product comprising machine executable instructions for execution by a processor (120) controlling a medical instrument (100, 400, 500, 600), wherein the medical instrument comprises an activity measurement system (106) configured for measuring brain activity data (138) from a subject (102) and a stimulus presentation system (108) configured for providing sensory stimulus to the subject, wherein execution of the machine executable instructions causes the processor to:
- control (200) the stimulus presentation system with a set of entries (136) from a stimulus reinforcer database to repeatedly provide sensory stimulus to the subject, wherein the stimulus reinforcer database comprises entries, wherein each entry comprises commands configured for controlling the stimulus presentation system to provide the sensory stimulus to the subject;
- control (202) the activity measurement system for performing the measurement of the brain activity data during each sensor stimulus;
- select (204) a chosen entry (140) from the set of entries using the brain activity data; and
- store (206) the chosen entry in the memory.

15. A method of operating a medical instrument (100, 400, 500, 600), wherein the medical instrument comprises an activity measurement system (106) configured for measuring brain activity data from a subject and a stimulus presentation system (108) configured for providing sensory stimulus to the subject, wherein the method comprises:
- controlling (200) the stimulus presentation system with a set of entries (136) from a stimulus reinforcer database (134) to repeatedly provide sensory stimulus to the subject, wherein the stimulus reinforcer database comprises entries, wherein each entry comprises commands configured for controlling the stimulus presentation system to provide the sensory stimulus to the subject;
- controlling (202) the activity measurement system for performing the measurement of the brain activity data during each sensor stimulus;
- selecting (204) a chosen entry (140) from the set of entries using the brain activity data; and
- storing (206) the chosen entry in the memory.
